# EUROPEAN PATENT SPECIFICATION

(11) **EP 1 874 378 B1**
(45) Date of publication and mention of the grant of the patent: **11.03.2009**
(21) Application number: 06727995.0
(22) Date of filing: 21.04.2006
(51) Int. Cl.: A61M 5/42, A61M 5/32, A61B 5/103, A61B 8/08

(54) **CANNULA INSERTING SYSTEM HAVING TISSUE ANALYSIS MEANS**
KANÜLENEINFÜHRSYSTEM MIT GEWEBEANALYSEMITTELN
SYSTEME D'INSERTION DE CANULE

(30) Priority: 22.04.2005 EP 05300309
(43) Date of publication of application: 09.01.2008
(73) Proprietor: Koninklijke Philips Electronics N.V., 5621 BA Eindhoven (NL)
(72) Inventor: NEERKEN, Sieglinde, Société Civile SPID, F-75008 Paris (FR); DE VREEDE, Frederikus, Société Civile SPID, F-75008 Paris (FR); HEKKENBERG, Robertus, Société Civile SPID, F-75008 Paris (FR); LUCASSEN, Gerhardus, Société Civile SPID, F-75008 Paris (FR); COHEN-SOLAL, Eric, Société Civile SPID, F-75008 Paris (FR); COHEN-BACRIE, Claude, Société Civile SPID, F-75008 Paris (FR); BALASUNDARA, Raju, Société Civile SPID, F-75008 Paris (FR); GEERLIGS, Marion, Société Civile SPID, F-75008 Paris (FR); EIKELENBERG, Nicole, Société Civile SPID, F-75008 Paris (FR); SCHWACH, Carole, Société Civile SPID, F-75008 Paris (FR)
(74) Representative: Schouten, Marcus Maria
(86) International application number: PCT/IB2006/051236
(87) International publication number: WO 2006/111939

(56) References cited:
- EP-A2- 0 190 719
- US-A1- 2003 233 046
- US-A1- 2004 236 224
- US-A1- 2005 027 185
- US-B1- 6 443 928
- VOGT M ET AL: "HIGH FREQUENCY ULTRASOUND FOR HIGH RESOLUTION SKIN IMAGING HOCHFREQUENTER ULTRASCHALL FUER DIE HOCHAUFLOESENDE ABBILDUNG DER HAUT" FREQUENZ, SCHIELE UND SCHON, BERLIN, DE, vol. 55, no. 1/2, 1 January 2001 (2001-01-01), pages 12-20, XP001003246 ISSN: 0016-1136
- DHAWAN A P ET AL: "Knowledge-based Color And Texture Analysis Of Skin Image" ENGINEERING IN MEDICINE AND BIOLOGY SOCIETY, 1990., PROCEEDINGS OF THE TWELFTH ANNUAL INTERNATIONAL CONFERENCE OF THE IEEE PHILADELPHIA, PA, USA 1-4 NOV. 1990, NEW YORK, NY, USA,IEEE, US, 1 November 1990 (1990-11-01), pages 1289-1290, XP010036223 ISBN: 0-87942-559-8

## Description

The present invention relates to the field of cannulation, hence to the insertion of a cannula or needle into the vascular system of a person or an animal.

Insertion of a cannula or a needle into a person's vascular system is an everyday task for physicians and has to be performed with high accuracy and care. Therefore, medical personnel has to be highly skilled for such tasks as blood withdrawal, drug delivery or infusions. The physician has to find an appropriate blood vessel and to introduce a distal end of a cannula or a needle with a very high precision in order to prevent the generation of hematoma or effusions. Depending on the vascular system of a person even a highly skilled and experienced physician may require several attempts to insert a needle or a cannula at a suitable location into a blood vessel. Such multiple attempts of puncturing are rather painful and cause appreciable patient discomfort. Moreover, such multiple attempts are also rather time intensive, which is disadvantageous especially in emergency situations.

There exist various devices and systems providing needle or cannula guiding and that assist a physician for inserting the cannula or needle in the vascular system of a patient.

US 2004/0236224 A1 discloses a system for the cannulation of blood vessels. The systems uses two ultrasound, linear transducer arrays to image blood vessels. The cannula is manually inserted under the guidance of the obtained image.

It is an object of the invention to provide a cannula inserting system providing an increased safety.

Another object of the invention is that a cannula inserting system is provided which is suitable for fully automatic use or for the semi-automatic use by unskilled users which is safe in handling.

According to the present invention the above-mentioned objects are achieved by providing the features defined in the independent claims. Preferred embodiments according to the invention additionally comprise the features of the sub-claims.

The present invention provides a puncture system for inserting a needle or cannula into a blood vessel of a person or an animal and comprises location determination means providing at least one location of the blood vessel. The system further comprises processing means for determining a puncture location of the blood vessel, hence an optimal location of the blood vessel that is potentially suitable for a needle or cannula insertion. This puncture location is determined by making use of output signals of the location determination means. The puncture system further comprises tissue analysis means for determining whether the tissue in the proximity of the puncture location is suitable for punctuation. The tissue analysis means thus checks whether the puncture location suggested by the processing means is actually suitable for punctuation.

Typically, the location determination means are adapted to provide a plurality of geometric data of the blood vessel, which allows to determine parameters such as blood vessel diameter, blood vessel type (vein or artery), blood vessel size as well as a depth under the skin. Further, the location determination means effectively provide determination of the blood vessel's course. The processing means make an effective use of such geometric and location information of the blood vessel which allows to determine a puncture location with a high accuracy and reliability that finally allows to minimize a danger of injury of a vessel wall. Consequently generation or severity of bleeding, hematoma or inflammation can be reduced to a minimum. Also by effective usage of obtained geometric and location data of the blood vessel, multiple attempts for a needle or cannula insertion can be prevented, because the reliable and accurate inspection of the blood vessel prior to insertion of the needle or cannula nearly guarantees that the needle or cannula can be correctly inserted or introduced into the vascular system with a single attempt. In particular, in emergency situations it is obvious, that this guided puncture is highly advantageous compared to an entirely manual cannulation.

The location determination means comprise optic and/or acoustic or opto-acoustic detection or signaling means and corresponding processing means that are adapted to perform a corresponding signal acquisition and signal processing for deriving relevant parameters for determination of the puncture location. The detection or signaling means may be realized as imaging and image processing means but may also be implemented as an acquisition system making use of e.g. Doppler based techniques that do not require image acquisition and respective image processing.

Typically, the location determination means are realized by making use of techniques, such as Near infrared imaging, Optical Coherence Tomography, Photo Acoustic Imaging or Ultrasound Techniques. In particular, Ultrasound techniques, Optical Coherence Tomography and Photo Acoustic Imaging provide inspection and analysis of the blood vessel and allow for a precise acquisition of blood vessel related data, even if the blood vessel is located at an appreciable depth under the skin of the person. Other techniques, that might be based on Doppler signals, like Doppler Ultrasound or Doppler Optical Coherence Tomography that are adapted to locate a flowing or streaming liquid, such as blood flowing in a blood vessel, in principle also allow a precise and reliable location determination of a blood vessel underneath the skin surface. Also, combinations of Doppler- based and imaging based signal acquisition techniques might be implemented.

Even if the processing means provide a reliable determination of the puncture location on the basis of the blood vessel parameters, in some instances, it must be noted that the suitability of the puncture location may not depend only on the above cited blood vessel parameters. Other parameters such as parameters of the tissue close to the blood vessel may also play a role and ought to be taken into account in some instances. Thus, tissue analysis means determines tissue parameters which indicate whether the tissue in the proximity of the puncture location is suitable for punctuation. This means that the processing means suggests a possible location for puncturing, and that the tissue analysis means checks whether this location is suitable for punctuation as far as the tissue is concerned.

It is however also possible that in a first step the tissue analysis means checks whether the skin is suitable for punctuation, and that in a second step the area of the skin deemed to be suitable is analyzed by the location determination means in order to determine a puncture location.

In an exemplary embodiment the tissue analysis means is adapted to analyse the skin surface, and more specifically to measure or analyze the melanin concentration of the skin. This measurement is performed in the proximity of the puncture location which has been determined by the processing means. In this way moles can be detected as they show a higher concentration of melanin in comparison to the surrounding skin. Moles however should not be used for puncturing, thus that the tissue analysis means avoid puncturing moles which increases the safety of the puncturing system.

In another exemplary embodiment the tissue analysis means is adapted to analyze the skin color and/or the brightness of the skin. This can be done by optical means which illuminate the skin and measure the remitted radiation dependent on the wavelength of the radiation. Analyzing the absorption profile thus acquired enables the tissue analysis means to detect skin irregularities such as lesions, scars, wounds, skin rash or the like not suitable for cannulation. These parts of the skin are normally unsuitable for puncturing, such that avoiding these portions of the body increases the safety of the puncturing system even further.

In a further embodiment of the invention the tissue analysis means are adapted to analyze the microstructure of the skin.

A "close-up" image of the skin surface for the analysis of the microstructure can be obtained in different ways. One possibility to analyze skin surface microstructure is to perform parallel polarized imaging with a broad spectral light source (white light). A second possibility is to make a UV photography or to make use of auto-fluorescence. However, already with a simple solution like close-up photography/imaging, or even with a flat bed scanner, the microstructure is visible. If a scar or wound is present the microstructure will be absent or have a different pattern. As with the color and brightness analysis the microstructure at the insertion site can be compared to that of the surrounding tissue to increase the sensitivity.

In a further embodiment the location determination are adapted to check whether the blood vessel is properly orientated with respect to the image plane of the location determination means and/or the orientation of the cannula.

According to another embodiment the puncture system has fastening means for fixing the needle or cannula with respect to the puncture system. The fastening means are moveable with respect to an inserting direction and at least with respect to a second direction that is substantially non-parallel to the inserting direction.

The inserting direction is typically given by the alignment of the needle or cannula and determines an angle at which the needle or cannula is intended to emerge the vascular system. Further, the fastening means are also moveable along at least a second direction, such as e.g. a direction being substantially parallel to the surface of the skin of the person or animal. Hence, the needle or cannula is moveable with respect to the location determination means as well as with respect to a blood vessel.

It is however also possible that a system comprising of the localisation determination means and the cannula is a fixed system where the components have a fixed spatial relationship to each other, and that this system is moveable as a whole with respect to the blood vessel.

Furthermore, the fastening means provide manual movement and alignment of the needle or cannula and provide manual insertion of the distal end of the needle or cannula into the blood vessel at the puncture location. In essence, this provides semi automated insertion of the needle or cannula. The puncture system autonomously allocates and determines a suitable puncture location and guides an operator to insert the needle or cannula at the specified location into the blood vessel. The actual insertion is performed operator supported. The force required for inserting the needle or cannula into the blood vessel is then provided by the operator, thus guaranteeing a maximum of sensitivity during needle or cannula insertion.

For instance, the location determination means and the processing means serve to locate and to identify a blood vessel and to determine the puncture location. However the needle or cannula insertion may not be performed in a completely automated but in a way that is at least partially controlled by the operator. For example, the puncture system may direct the needle or cannula along the inserting direction and may also move the cannula to the inserting position whereas the actual needle insertion, i.e. translation of the cannula or needle along the inserting direction is performed by the operator.

In a further embodiment the location determination means is adapted to check whether the blood vessel is properly orientated with respect to the cannula. The angle between the cannula and the blood vessel is dependent on the depth of the vessel, length of the needle, and other parameters. In blood vessels in the arm the angle will in most cases be between 15° and 30°. For catheter insertion into the central vein very large angles with angles larger than 45 degrees are required due to the deep location of the blood vessel.

According to a further embodiment, the location determination means is not only adapted to detect and to identify a blood vessel underneath the skin surface but also provide location determination of the cannula's distal end. Hence, by means of the location determination means, it can be precisely checked whether the cannula or needle has been correctly inserted into the vascular system of the person or animal. Additionally, the puncture system features respective indication means, that are adapted to indicate whether the cannula or needle has been correctly inserted into the blood vessel by the puncture system.

According to a further embodiment, the location means is further adapted to track the location of the needle's or cannula's distal end during insertion of the needle or cannula. The puncture system further has control means for controlling the movement of the needle or cannula in response to the tracking of the needle's or cannula's distal end. In this way, the puncture system is provided with a feedback allowing to monitor and to check whether the distal end of the needle or cannula is correctly inserted. This functionality effectively represents a safety mechanism of the puncture system and helps to prevent that despite of an accurate inspection of the blood vessel the cannula might be incorrectly introduced, which may have serious consequences for the person's or animal's health.

Typically, the location determination means provides course and location determination of the blood vessel as well as tracking of the needle's or cannula's distal end at a sufficient repetition rate that allows for fast reaction in case that the cannula introduction deviates from a determined path or schedule. Also, the location determination means allows to check whether the distal end of the needle or cannula has been inserted correctly into the persons vascular system. Hence, the location determination means not only provides a control mechanism during needle or cannula insertion but also allows to check the final position of the needle or cannula after the intra vascular inserting has been terminated.

It is also possible to monitor and follow the position and/or movement of the blood vessel during insertion of the cannula or needle. This should also provide enough information for a closed loop system and is easier in implementation in comparison to the solution of the last paragraph. If it is known where the needle has to end up and if the insertion parameters are known, the location of the blood vessel can be monitored during insertion. The insertion is not successful if the blood vessel moves or does not stay in place.

According to an embodiment, the puncture system further comprises actuation means that is adapted to autonomously move the fastening means into an inserting position and that is further adapted to insert the distal end of the needle or cannula into the blood vessel at the puncture location. In this embodiment the invention provides an entirely automated needle or cannula insertion. Hence, the inventive puncture system provides a location determination of the blood vessel, an autonomous alignment and movement of the cannula to an inserting position and inserting direction and finally an automated inserting of the cannula into the blood vessel at a location determined by the puncture system itself.

In this sense the inventive puncture system provides an entirely automated insertion of a needle or cannula into a person's or animal's blood vessel, which is applicable to e.g. blood sampling or blood withdrawal, drug medication or infusion, blood transfusion, general catheter insertion and dialysis. The entire process of locating of a blood vessel, determining of a puncture location as well as mechanically shifting and aligning the needle or cannula and finally inserting of the needle or cannula can be performed without any user interaction, allowing to execute the above mentioned tasks in a highly automated manner that may even be performed by unskilled or low-skilled medical personnel.

According to a further embodiment, the actuation means are manually controllable by an operator for manual insertion of the cannula by the operator. Even though if implemented as an autonomous system, the puncture system also allows for a partially automated insertion of a needle or cannula.
The needle or cannula may be used for blood withdrawal and/or drug infusion and/or blood transfusion, and/or catheter insertion and/or dialysis applications. Hence, the invention can be universally applied to various different medical purposes that require insertion of a needle or cannula into a vascular system of a person. Respective fastening means for fixing the needle or cannula are typically realized by making use of a modular concept allowing for a quick and secure adaptation of the needle or cannula inserting system to a multitude of different purposes.

In another aspect, the invention provides a computer program product which is executable by processing means of the puncture system and is further operable to perform a determination of at least one blood vessel parameter and at least one tissue parameter by processing of the output of location determination means and the output of the tissue analysis means. The computer program product is inherently adapted to recognize and to identify a blood vessel and the surrounding tissue. It is further enabled to exploit the blood vessel recognition and identification for acquiring the at least one relevant blood vessel parameters. This at least one blood vessel parameter is at least representative of a blood vessel's location underneath the surface of the skin. Preferably, a plurality of blood vessel parameters representing blood vessel type (e.g. vein or artery), blood vessel size, blood vessel diameter as well as a course of the blood vessel, geometry of the blood vessel and depth underneath the surface of the skin can be precisely determined.

The computer program product is further operable to determine a puncture location of the blood vessel by processing of the at least one blood vessel parameter. Preferably, by making use of a plurality of blood vessel parameters, the computer program product is operable to perform an optimization procedure in order to determine an optimal puncture location of the blood vessel.
Furthermore the computer program product is enabled to analyze the insertion site below and above the skin surface to check whether it is suitable for punctuation. For that purpose it is adapted to determine and process tissue parameters such as the melanin concentration, the color of the skin, the brightness of the skin, the orientation of the cannula with respect to the blood vessel, the orientation of the blood vessel with respect to the image plane, the orientation of the cannula with respect to the image plane, and similar parameters.

These and other aspects of the invention will be apparent from and elucidated with reference to the embodiments described thereafter. It should be noted that the use of reference signs shall not be construed as limiting the scope of the invention.
Fig. 1 illustrates a schematic block diagram of the puncture system,
Fig. 2a shows a measurement system for examining the targeted insertion location,
Fig. 2b shows the absorption profile obtained with the measurement system of Fig. 2a,
Fig. 3a shows a measurement system for examining the targete insertion location in the case of a mole at the puncture location,
Fig. 3b shows the absorption profile obtained with the measurement system of Fig. 3a,
Fig. 4 shows possible orientations of the cannula in cases in which the blood vessel is perpendicular to the image plane,
Fig. 5 shows possible orientations of the cannula in cases in which the blood vessel is parallel to the image plane,
Fig. 6 shows a dual imaging of a blood vessel with a single planar probe having two image planes,
Fig. 7 shows a dual imaging of a blood vessel with two one-dimensional probes arranged parallel to each other,
Fig. 8 shows methods to acquire three-dimensional blood vessel images,
Fig. 9 shows a schematic illustration of puncture location and inserting position determined by the puncture system.

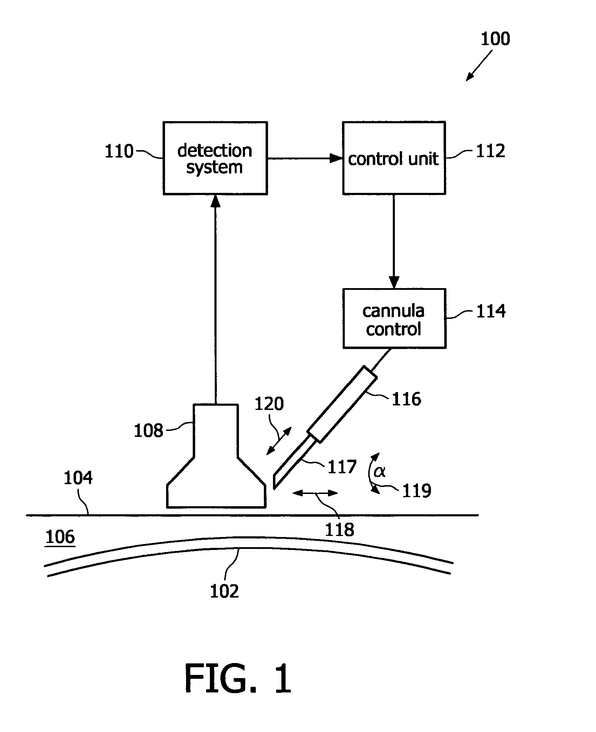

Fig. 1 shows a schematic block diagram of the puncture system 100. The puncture system 100 has an acquisition module 108, a detection system 110, a control unit 112, a cannula control 114 as well as a cannula mount 116. The cannula 117 itself can be rigidly attached to the cannula mount 116 that represents fastening means for fixing the cannula and means for moving and aligning the cannula 117 as controlled by the cannula control unit 114. The cannula 117 and the cannula mount 116 can be moved along the inserting direction 120 as well as along direction 118 that is substantially parallel to the surface of the skin 104. In principle direction 118 can be any direction in the plane parallel to the skin surface. Typically, the cannula 117 and the cannula mount 116 are moveable by means of the cannula control 114 in all three spatial directions. Also, the angle α 119 between the inserting direction 120 and the surface of the skin 104 may be arbitrarily modified by means of the cannula control 114 in a way that is determined by means of the detection system 110 and the control unit 112.

Fig. 1 shows application of the puncture system to a person by means of a cross sectional illustration of the person's skin 104. Underneath the surface of the skin 104 there is located a blood vessel 102 that is surrounded by tissue 106. When the puncture system 100 is attached to the skin 104 of the person, the acquisition module 108 is adapted to acquire optical, opto-acoustic or acoustic data from the tissue 106 and the blood vessel 102 that allows to classify at least one blood vessel parameter, such as location of the blood vessel, diameter of the blood vessel, size of the blood vessel, depth underneath the surface of the skin 104, geometry of the blood vessel, blood flow or similar parameters.

The acquisition module 108 may be realized by means of Ultrasound, Near-infrared imaging, Optical Coherence Tomography, Doppler Ultrasound, Doppler Optical Coherence Tomography or Photo Acoustic techniques that allow to generate a signal providing identification of the blood vessel 102. Signals acquired by the acquisition module 108 are provided to the detection system 110, which in turn generates a signal of the blood vessel 102. Hence, detection system 110 as well as acquisition module 108 are coordinated in a sense that the detection system 110 is suitable to perform signal processing of signals obtained from the acquisition module 108. By making use of optical, opto-acoustic or ultrasound detection, the blood vessel 102 may be precisely located even at an appreciable depth underneath the surface of the skin 104. Additionally or alternatively also Doppler techniques may be applied including e.g. Doppler Ultrasound techniques allowing for detection of e.g. blood flow in the blood vessel 102. Also, Doppler Optical Coherence Tomography might be correspondingly applied.

Acquisition of location data, geometric data as well as data related to the course of the blood vessel 102, may also be obtained without an imaging of the blood vessel. Therefore, the imaging system 110 does not necessarily have to provide a visual image. Instead the imaging system 110 may be enabled to directly extract blood vessel parameters from the signals acquired by the acquisition module 108. Hence, extraction of blood vessel parameters may be performed by means of the detection system 110 or by the control unit 112.

The control unit 112 has a processing unit that is enabled to process the data obtained from the detection system 110. Depending on the type of data provided by the detection system 110, the processing unit of the control unit 112 may further process blood vessel parameters in order to extract required blood vessel parameters from a signal of the blood vessel 102. Furthermore, the control unit 112 is enabled a tissue analysis to check whether the tissue in the proximity of the puncture location is suitable for punctuation.

The control unit 112 serves to process the blood vessel parameters in order to find and to determine a puncture location of the blood vessel 102 that is ideally suited for an insertion of the cannula 117. In a basic embodiment this puncture location may be determined with respect to location and course of the blood vessel 102. More sophisticated implementations further account for the vessel geometry in the vicinity of an intended puncture location as well as vessel diameter and depth underneath the surface of the skin 104.

Typically, the puncture location may be determined as a result of an optimization procedure taking into account all kinds of blood vessel parameters. For instance, the optimization procedure that is typically performed by means of the processing unit of the control unit 112 may specify, that a puncture location must not be in the vicinity of a branch or junction of a blood vessel 102. Further, a puncture location may require a certain diameter of the blood vessel 102. Also, the puncture location may be determined with respect of a smallest possible depth of the blood vessel 102 underneath the surface of the skin 104. Additionally, the control unit may also determine the inserting direction 120 specifying at which angle α 119 the cannula 117 has to be introduced into the skin 104 and the tissue 106.

Having determined the puncture location, the control unit 112 is further adapted to specify an inserting position for the cannula 117. The inserting position specifies a position as well as an alignment or direction of the cannula 117 from which the cannula 117 has to be shifted along the inserting direction, i.e. the direction coinciding with the longitudinal direction of the cannula, in order to impinge into the blood vessel at the determined puncture location with its distal end.

After specifying a puncture location tissue analysis means check whether the tissue 106 surrounding the insertion position 126 is suitable for puncturing. In the alternative, a reverse order is chosen: in a first step the skin surface is analyzed, and if this is ok, a blood vessel is determined. The tissue analysis means might be separate means or are provided as an additional functionality of the control unit 112. For the latter case the firmware the control unit 112 has to be supplemented accordingly. Control unit 112 then needs to analyze the output of the detection system 110 adapted to provide an measurement of the puncture location 124.

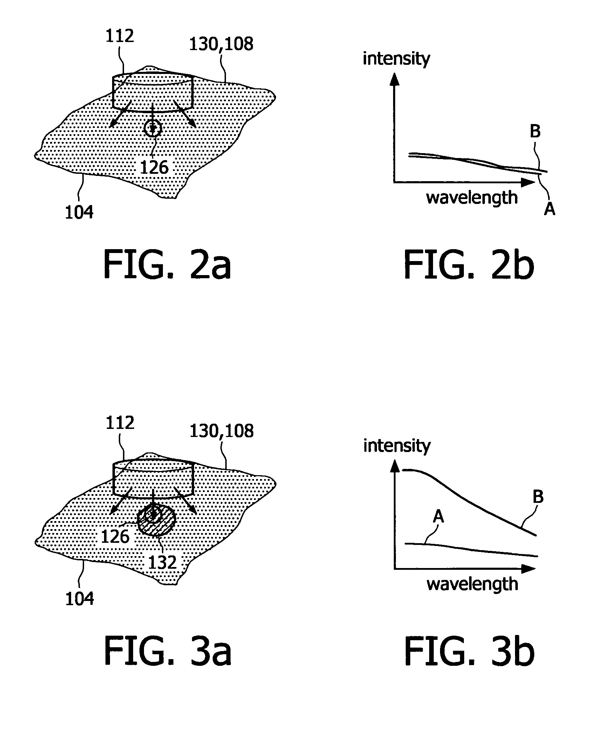

One possibility to analyze the tissue 106 in the proximity of the insertion position 126 is skin surface analysis by illuminating the proximity of the insertion position 126 with electromagnetic radiation in the visible part of the spectrum and by detecting the remitted light. This is shown in Fig. 2a, in which a measurement unit 130 illuminates the skin 104 in the proximity of the insertion position 126. For that purpose wavelengths between 400 nm and 800 nm are used. The measurement unit 130 may be a separate entity adjacent to the acquisition module 108, or may form an integral part of the acquisition module 108.

Fig. 2b shows the absorption profile both for the insertion position 126 predetermined by the processing means 112, and for the surrounding tissue. The intensity of the remitted light is plotted versus the wavelength detected by the measurement unit 130. The curves are indistinguishable which indicates that the melanin concentration is homogenously distributed. In other words the predetermined insertion position 126 is not located within a mole.

Fig. 3a shows the case corresponding to Fig. 2a in which the predetermined insertion position 126 is located within a mole 132. In this case the absorption profile of Fig. 3b shows low values of remitted light for the surrounding tissue (curve A), but a high intensity at the predetermined insertion position 126 (curve B). As can be derived from Fig. 3b, the absorption spectrum of melanin is very broad. Generally, the absorption profile of melanin does not always show a clear absorption maximum with a gaussian peak.

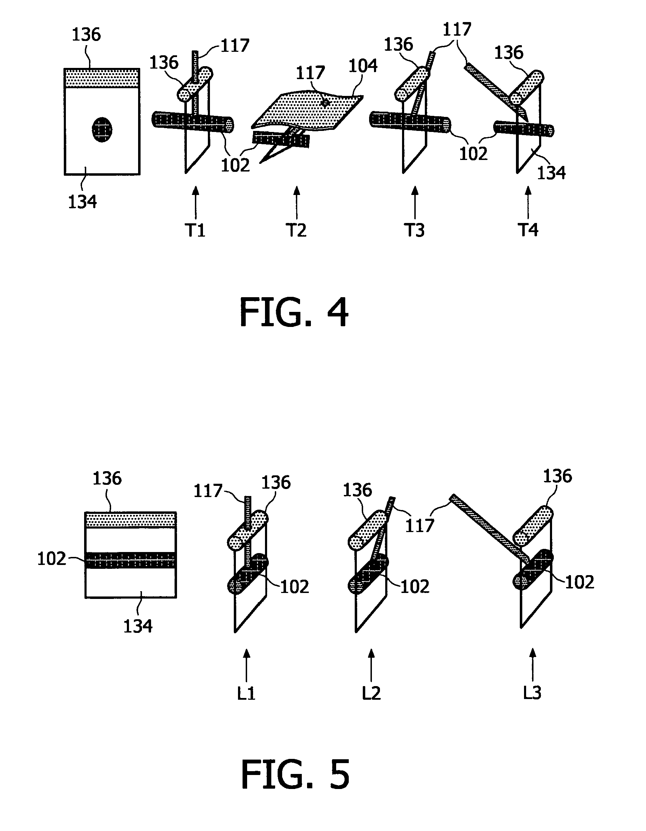

Fig. 4 shows possible orientations of the cannula 117 in cases in which the blood vessel 102 is perpendicular to the image plane 134 (transverse view) obtained by the probe 136 of the acquisition module 108, such that two-dimensional images are obtained.

In case T1 the cannula 117 penetrates both the probe 136 of the acquisition module 108 and the blood vessel 102 and lies within the image plane 134. No elements of the probe 136 are present at the cannula 117 position. This orientation is unwanted as no signal can be acquired from the distal end of the cannula 117. Furthermore, the angle between the blood vessel 102 and the cannula 117 is too large.

Case T2 corresponds to case T1, whereby the image plane 124 is tilted clockwise. Now the angle between the blood vessel 102 and the cannula 117 is closer to the desired range of 15° to 30°, but still no signal can be acquired from the distal end of the cannula 117.

In case T3 the complete cannula 117 is visible in the image plane 134 and its movement can be followed during insertion. In this case the needle is inserted perpendicular to the orientation of the blood vessel, which requires very high precision and lowers the chance of keeping the distal end of the needle inside the vessel. In clinical practice the needle is usually inserted in the direction of the blood vessel.

In case T4 the cannula 117 intersects the image plane 134. In this case only a cross-section of the distal end of the cannula is visible in the image plane 134. In case T4 only the distal end of the cannula 117 is imaged. In this case the angle between the blood vessel 102 and the cannula 117 is closer to the desired range of 15° to 30 and the needle is inserted in the direction of the vessel. T3 and T4 are the most favourable cases in the transverse view.

Fig. 5 shows possible orientations of the cannula 117 in cases in which the blood vessel 102 is parallel to the image plane 134 of probe 136 (longitudinal view).

In case L1 the cannula 117 penetrates the probe 136 as well as the blood vessel 102. No elements of the probe 136 are present the cannula 117 position. With this orientation no signals directly from the distal end of the cannula 117 will be detected and the exact position of the distal end cannot be followed in the image.

In case L2 the complete cannula 117 is visible in the image plane 134 and its movement can be followed during insertion. This is the most favourable orientation in the longitudinal view. In case L3 the cannula or needle 117 is inserted perpendicular to the blood vessel 102. The needle tip is visible in the image plane 134. The perpendicular configuration is more difficult to achieve and the exact position of the distal end cannot be followed in the image.

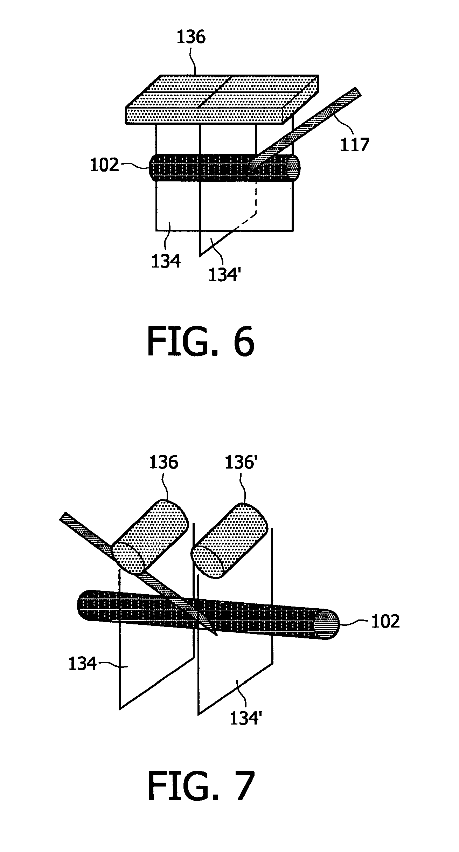

It is also possible to use a combination of two probes with two image planes not being parallel to each other, e.g. being perpendicular to each other. Fig. 6 shows such a case when a single probe 136 is used which can measure in two non-parallel directions with image planes 134, 134' respectively. The cannula 117 or needle is completely visible and its movement can be followed during insertion.

Fig. 7 shows the case of at least two one-dimensional probes 136, 136' aligned parallel to each other, the probes 136, 136' having two image planes 134 and 134' respectively. This is equivalent to two configurations T4 of Fig. 4. In this case two intersections of the cannula 117 with the image planes 134, 134' are obtained. The two intersections can be approximated to be two points, such that the geometrical location of these two points accurately define the orientation of the cannula 117. This is achieved with two one-dimensions probes 136, 136'.

The most complete information of the puncture location can be obtained by a three-dimensional (3D) image acquisition. In this case the orientation of the blood vessel can be inferred more precisely as the 3D image makes sudden bifurcations and changes of its depth under the surface of the skin visible. With the 3D information the precision of cannula insertion into the blood vessel can be higher and cases of unsuccessful insertion can be reduced. Furthermore, the depth of the blood vessel below the skin surface can be derived in a certain volume which is particularly important in an automatic operation mode of the puncture system.

Two methods can be used to realize a 3D image of the puncture location.

A first possibility is to move the probe 136 during a measurement and to reconstruct a 3D image from the individual two-dimensional (2D) images. In Case 3D-1 the probe 136 is moved from left to right or vice versa as indicated by the arrows. In case 3D-2 the cylindrically shaped probe 136 is rotated around its symmetry axis as indicated by the double arrow. In both cases cross-sectional views of the cannula 117 are visible in the image plane 134 during the movement of the probe 136. The position of the cannula 117 can be followed during its movement.

In case 3D-3 probe 136 is rotated around an axis being substantially perpendicular to the skin surface. to the blood vessel 102. This is indicated by the double arrow. Depending on the position of the probe 136 the visibility of the cannula 117 changes from a cross-sectional view to a longitudinal view. The position of the cannula 117 can be followed during its movement.

A second possibility is to use a non-moving probe 136 being able to measure in two non-parallel directions, confer case 3D-4. In this case the position of the cannula 117 and its orientation is always visible and its movement can be followed during puncturing.

Summarizing all the possibilities given above a 2D imaging is basically simpler to implement and needs less hardware resources.

Basically 2D imaging methods are preferred which enable to follow the cannula and/or the blood vessel during the punctuation in order to have the possibility to correct the progress of propagation of the cannula.

3D imaging has the advantage that the course of the blood vessel can be derived such that bifurcations do not render it impossible to puncture the blood vessel. Furthermore the depth of the blood vessel below the skin surface can be acquired, such that the corresponding depth of the cannula can be chosen with higher precision. This stems from the fact that 3D imaging provides a global overview of a certain volume of the tissue such that the course (depth, location, size) of the vessel is followed in the whole volume and not at a single position.

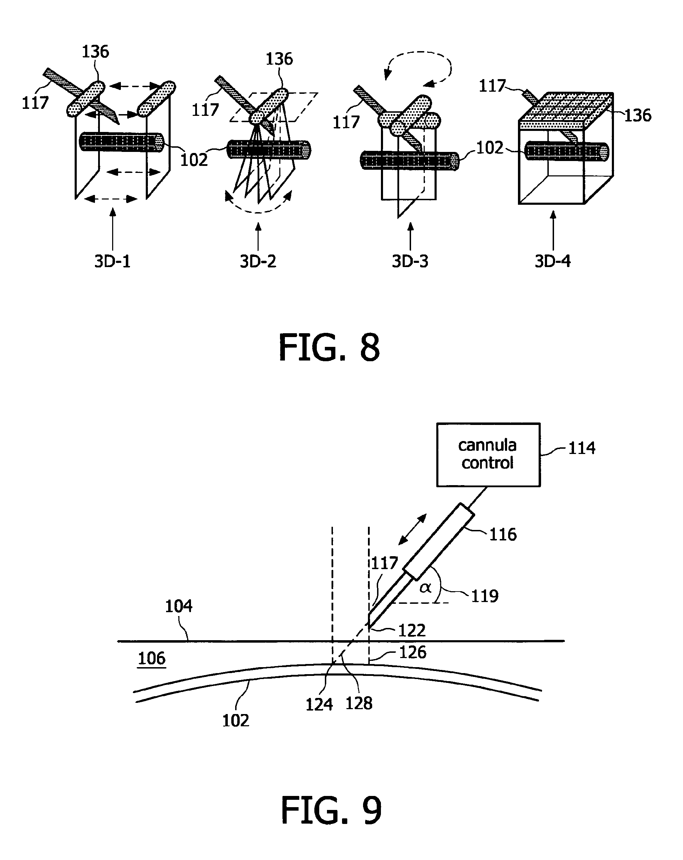

Fig. 8 shows a schematic illustration of puncture location 124 and the inserting position 126 that are determined by the puncture system. Similar to Fig. 1 also a cross sectional view of a person's or animal's skin is shown. The puncture system 100 determines the puncture location 124 of the blood vessel 102 by making use of blood vessel parameters that were obtained by means of the acquisition module 108 and corresponding detection and processing means. Here, the blood vessel 102 shows a uniform diameter and the puncture location 124 is determined by a position of the blood vessel 102 that is closest to the surface of the skin 104. This puncture location 124 may also be chosen by an experienced physician for manually inserting the cannula into the blood vessel 102. Hence, the inventive puncture system aims to determine a puncture location that provides a minimum of discomfort and pain as well as a minimum of danger of injury to the vessel wall, which may prevent potential severe consequences for the health status of the person.

Furthermore, the control unit 112 may also determine an optimal insertion angle α 119 that determines the insertion direction 120 of the cannula 117. Since the cannula 117 is typically introduced at a non-perpendicular angle with respect to the surface of the skin 104, the point of penetration through the skin 104 and the puncture location 124 typically describe an insertion path 128 for the cannula 117 that coincides with the inserting direction 120. Before advancing the cannula 117 into the skin 104, it has to be moved to the inserting position 126 featuring a lateral displacement from the puncture location 124. In this context, lateral displacement is to be interpreted as a displacement in the plane of the surface of the skin 104. For instance, the inserting position 126 may be determined as the position where the distal end of the cannula 122 coincides with the insertion path 128.

Insertion of the needle is possibly performed as a two step process, wherein the first step is given by moving the cannula 117 to the inserting position 126 by means of the cannula control unit 114. As soon as the inserting position 126 has been reached by the distal end of the cannula 122, the second step of advancing and inserting the cannula 117 into the skin 104, the tissue 106 and finally into the puncture location of the blood vessel 102 is initialized. Advancing and inserting of the cannula 117 is controlled by means of the acquisition module 108 and the control unit 112 in order to correct the movement of the cannula 117 during the insertion process. However, as soon as the acquisition module 108 detects, that the distal end of the cannula 122 has penetrated through the surface of the skin 104, a lateral movement of the cannula by means of the cannula control 114 is disabled for preventing severe injury of the skin 104 and the tissue 106.

Additionally, the puncture system 100 may be provided with a release module in order to manually control the insertion of the cannula 117. Then, a user of the puncture system may manually trigger the advancing of the cannula 117 along the inserting direction 120 and may manually control whether the inserting position 126 and/or the puncture location 124 determined by a puncture system are reasonable for inserting a cannula.

## Claims

1. A puncture system (100) for inserting a cannula (117) into a blood vessel (102) of a person or animal comprising:
a) location determination means (108, 110) for determining at least one location of the blood vessel,
b) processing means (112) for determining a puncture location (124) of the blood vessel depending on the output of the location determination means,
c) tissue analysis means (112) for determining whether the tissue in the proximity of the puncture location (124) is suitable for punctuation.

2. The system according to claim 1, **characterized in that** the tissue analysis means is adapted to analyze one or more of the followings: the skin surface, the melanin concentration of the skin, the color of the skin, and the microstructure of the skin.

3. The system according to claim 1, **characterized in that** the location determination means is adapted to check whether the cannula is properly orientated with respect to the image plane (134) of the location determination means and/or the orientation of the blood vessel.

4. The system according to claim 1, **characterized in that** the system further comprises fastening means (116) for fixing the cannula, said fastening means being moveable along an inserting direction (120) and at least a second direction (118) being substantially non-parallel to the inserting direction, for insertion of the distal end (122) of the cannula into the blood vessel at the puncture location.

5. The system according to claim 1, **characterized in that** the location determination means is further adapted to determine the location of the cannula's distal end (122).

6. The system according to claim 1, **characterized in that** the location determination means is adapted to follow the blood vessel position during insertion.

7. The system according to claim 1, **characterized in that** the system comprises actuation means being adapted to autonomously move the fastening means into an inserting position (126) and to insert the distal end (122) of the cannula into the blood vessel at the puncture location.

8. The system according to claim 1, **characterized in that** the cannula (117) is applicable to blood withdrawal and/or drug infusion and/or blood transfusion, and/or catheter insertion and/or dialysis applications.

9. The system according to claim 1, **characterized in that** the system is adapted to autonomously perform the insertion of the cannula's distal end (122) into the blood vessel (102) in response of detecting that the cannula (117) is at the insertion position (126).

10. The system according to claim 1, **characterized in that** the system is adapted to autonomously perform the insertion of the cannula's distal end (122) into the blood vessel (102) in response of detecting the puncture location (124) and/or in response of detecting that the blood vessel does not move.

11. A computer program product, comprising a computer readable medium, having thereon computer program code means, which, when said program is loaded, make the computer execute the following steps:
a) determining a blood vessel parameter of a blood vessel by processing the output of location determination means, the at least one blood vessel parameter being representative of the blood vessel's location,
b) determining a puncture location (124) of the blood vessel by processing of the at least one blood vessel parameter,
c) analyzing a tissue parameter of the tissue in the proximity of the puncture location (124) by processing the output of tissue analysis means (112), said tissue parameter being representative of the suitability of the puncture location for punctuation.

## Patentansprüche

1. Einstichsystem (100) zum Einführen einer Kanüle (117) in ein Blutgefäß (102) einer Person oder eines Tieres, wobei das Einstichsystem Folgendes umfasst:
a) Positionsbestimmungsmittel (108, 110) zum Bestimmen mindestens einer Position des Blutgefäßes,
b) Verarbeitungsmittel (112) zum Bestimmen einer Einstichstelle (124) des Blutgefäßes abhängig von der Ausgabe des Positionsbestimmungsmittels,
c) Gewebeanalysemittel (112) zum Ermitteln, ob das Gewebe in der Nähe der Einstichstelle (124) für den Einstich geeignet ist.

2. System nach Anspruch 1, **dadurch gekennzeichnet, dass** das Gewebeanalysemittel dafür eingerichtet ist, einen oder mehrere der folgenden Aspekte zu analysieren: die Hautoberfläche, die Melaninkonzentration der Haut, die Farbe der Haut und die Mikrostruktur der Haut.

3. System nach Anspruch 1, **dadurch gekennzeichnet, dass** das Positionsbestimmungsmittel dafür eingerichtet ist, zu prüfen, ob die Kanüle in Bezug auf die Bildebene (134) des Positionsbestimmungsmittels und/oder die Orientierung des Blutgefäßes korrekt ausgerichtet ist.

4. Verfahren nach Anspruch 1, **dadurch gekennzeichnet, dass** das System weiterhin Befestigungsmittel (116) zum Befestigen der Kanüle umfasst, wobei die genannten Befestigungsmittel entlang einer Einführungsrichtung (120) bewegbar sind und mindestens eine zweite Richtung (118) im Wesentlichen nicht-parallel zu der Einführungsrichtung verläuft, um das distale Ende (122) der Kanüle an der Einstichstelle in das Blutgefäß einzuführen.

5. System nach Anspruch 1, **dadurch gekennzeichnet, dass** das Positionsbestimmungsmittel weiterhin dafür eingerichtet ist, die Position des distalen Kanülenendes (122) zu ermitteln.

6. System nach Anspruch 1, **dadurch gekennzeichnet, dass** das Positionsbestimmungsmittel dafür eingerichtet ist, der Blutgefäßposition während der Einführung zu folgen.

7. System nach Anspruch 1, **dadurch gekennzeichnet, dass** das System Betätigungsmittel umfasst, die dafür eingerichtet sind, das Befestigungsmittel autonom in eine Einführungsposition (126) zu bringen und das distale Ende (122) der Kanüle an der Einstichstelle in das Blutgefäß einzuführen.

8. System nach Anspruch 1, **dadurch gekennzeichnet, dass** die Kanüle (117) für die Blutentnahme und/oder Arzneimittelinfusion und/oder Bluttransfusion und/oder für die Kathetereinführung und/oder für Dialyseanwendungen einsetzbar ist.

9. System nach Anspruch 1, **dadurch gekennzeichnet, dass** das System dafür eingerichtet ist, das Einführen des distalen Kanülenendes (122) in das Blutgefäß (102) autonom in Reaktion auf das Detektieren der Anwesenheit der Kanüle (117) an der Einführungsposition (126) durchzuführen.

10. System nach Anspruch 1, **dadurch gekennzeichnet, dass** das System dafür eingerichtet ist, die das Einführen des distalen Kanülenendes (122) in das Blutgefäß (102) in Reaktion auf das Detektieren der Einstichposition (124) und/oder in Reaktion auf das Detektieren der Unbeweglichkeit des Blutgefäßes autonom durchzuführen.

11. Computerprogrammprodukt, das ein computerlesbares Medium umfasst, auf dem sich Computerprogrammcodemittel befinden, die, wenn das genannte Programm geladen wird, den Computer zur Ausführung der folgenden Schritte veranlassen:
a) Ermitteln eines Blutgefäßparameters eines Blutgefäßes durch Verarbeiten der Ausgabe des Positionsbestimmungsmittels, wobei der mindestens eine Blutgefäßparameter für die Position des Blutgefäßes repräsentativ ist,
b) Ermitteln einer Einstichstelle (124) des Blutgefäßes durch Verarbeiten des mindestens einen Blutgefäßparameters,
c) Analysieren eines Gewebeparameters des Gewebes in der Nähe der Einstichstelle (124) durch Verarbeiten der Ausgabe des Gewebeanalysemittels (112), wobei der genannte Gewebeparameter für die Eignung der Einstichstelle zum Einstechen repräsentativ ist.

## Revendications

1. Système de ponction (100) destiné à insérer une canule (117) dans un vaisseau sanguin (102) d'une personne ou d'un animal comprenant :
a) un moyen de détermination d'emplacement (108, 110) pour déterminer au moins un emplacement d'un vaisseau sanguin,
b) un moyen de traitement (112) pour déterminer un emplacement de ponction (124) du vaisseau sanguin en fonction du résultat du moyen de détermination d'emplacement,
c) un moyen d'analyse du tissu (112) pour déterminer si le tissu à proximité des emplacements de ponction (124) est adapté pour ladite ponction.

2. Système selon la revendication 1, **caractérisé en ce que** le moyen d'analyse du tissu est adapté pour analyser un ou plusieurs des éléments suivants : la surface de la peau, la concentration en mélanine de la peau, la couleur de la peau, et la microstructure de la peau.

3. Système selon la revendication 1, **caractérisé en ce que** le moyen de détermination d'emplacement est adapté pour vérifier si la canule est correctement orientée relativement au plan de l'image (134) du moyen de détermination d'emplacement et/ou de l'orientation du vaisseau sanguin.

4. Système selon la revendication 1, **caractérisé en ce que** le système comprend en outre un moyen de fixation (116) pour fixer la canule, ledit moyen de fixation étant mobile le long d'une direction d'insertion (120) et au moins une seconde direction (118) sensiblement non parallèle à la direction d'insertion, pour l'insertion de l'extrémité distale (122) de la canule dans le vaisseau sanguin à l'emplacement de la ponction.

5. Système selon la revendication 1, **caractérisé en ce que** le moyen de détermination d'emplacement est en outre adapté pour déterminer l'emplacement de l'extrémité distale de la canule (122).

6. Système selon la revendication 1, **caractérisé en ce que** le moyen de détermination d'emplacement est adapté pour suivre la position du vaisseau sanguin pendant l'insertion.

7. Système selon la revendication 1, **caractérisé en ce que** le système comprend un moyen d'actionnement adapté pour déplacer de façon autonome le moyen de fixation dans une position d'insertion (126) et pour insérer l'extrémité distale (122) de la canule dans le vaisseau sanguin à l'emplacement de la piqûre.

8. Système selon la revendication 1, **caractérisé en ce que** la canule (117) peut être utilisée pour le prélèvement de sang et/ou la perfusion de médicaments et/ou la transfusion sanguine, et/ou l'insertion de cathéter et/ou les applications de dialyse.

9. Système selon la revendication 1, **caractérisé en ce que** le système est adapté pour effectuer de manière autonome l'insertion de l'extrémité distale de la canule (122) dans le vaisseau sanguin (102) en réponse à la détection que la canule (117) est dans la position d'insertion (126).

10. Système selon la revendication 1, **caractérisé en ce que** le système est adapté pour effectuer de manière autonome l'insertion de l'extrémité distale de la canule (122) dans le vaisseau sanguin (102) en réponse à la détection de l'emplacement de ponction (124) et/ou en réponse à la détection indiquant que le vaisseau sanguin ne bouge pas.

11. Progiciel informatique, comprenant un système lisible par ordinateur, sur lequel se trouve un système de codage de programme informatique, qui, lorsque ledit programme est chargé, permet à l'ordinateur d'exécuter les phases suivantes :
a) détermination d'un paramètre de vaisseau sanguin relatif à un vaisseau sanguin, en traitant le résultat du moyen de détermination d'emplacement, ledit paramètre de vaisseau sanguin étant représentatif de l'emplacement du vaisseau sanguin,
b) détermination d'un emplacement de ponction (124) du vaisseau sanguin en traitant ledit paramètre de vaisseau sanguin,
c) analyse d'un paramètre tissulaire du tissu à proximité de l'emplacement de ponction (124), en traitant le résultat du moyen d'analyse du tissu (112), ledit paramètre tissulaire étant représentatif du caractère adapté de l'emplacement de ponction pour ladite ponction.
